# EUROPEAN PATENT APPLICATION

(11) **EP 1 518 556 A1**
(43) Date of publication of application: **30.03.2005**
(21) Application number: 03736270.4
(22) Date of filing: 26.06.2003
(51) Int. Cl.: A61K 31/498, A61P 7/04, A61P 25/00, A61P 25/28, C07D 403/04

(54) **THERAPEUTIC AGENT FOR BRAIN HEMORRHAGE**

(30) Priority: 28.06.2002 JP 2002188919
(71) Applicant: YAMANOUCHI PHARMACEUTICAL CO. LTD., Tokyo 103-8411 (JP)
(72) Inventor: TERAI, Kazuhiro, c/o Yamanouchi Pharma. Co. Ltd., Tsukuba-shi, Ibaraki 305-8585 (JP); SUZUKI, Masanori, c/o Yamanouchi Pharma. Co. Ltd., Tsukuba-shi, Ibaraki 305-8585 (JP); SASAMATA, Masao, c/o Yamanouchi Pharma. Co. Ltd., Tsukuba-shi, Ibaraki 305-8585 (JP)
(74) Representative: Bates, Philip Ian
(86) International application number: PCT/JP2003/008128
(87) International publication number: WO 2004/002488

(57) **Abstract**

It was found that an AMPA receptor antagonist zonampanel or a salt or the like thereof has an effect to improve cerebral hemorrhage and neurological symptoms accompanied by cerebral hemorrhage and therefore is useful as an agent for treating cerebral hemorrhage.

## Description

### Technical Field

The present invention relates to an agent for treating cerebral hemorrhage, comprising [7-(1H-imidazol-1-yl)-6-nitro-2,3-dioxo-3,4-dihydroquinoxalin-1(2H)-yl]acetic acid or a salt thereof or a hydrate thereof as the active ingredient, which aims at controlling and improving symptoms accompanied by cerebral hemorrhage, and to its novel medical use as an agent for improving neurological symptoms accompanied by cerebral hemorrhage.

### Background of the Technique

It is said that cerebral hemorrhage is frequent in Japan in comparison with Europe and America, and the ratio of cerebral hemorrhage occupying general stroke is about 17% (*Stroke,* 19: 45 - 52, 1988).

Cerebral hemorrhage means bleeding generated in the brain parenchyma, and it includes ventricular hemorrhage but does not include subarachnoid hemorrhage, subdural hemorrhage and hemorrhagic infarction. It is known that cerebral hemorrhage is also generated by cerebral aneurysm rupture, rupture of intracranial arteriovenous malformation, cavernous angioma, head injury, amyloid angiopathy, taking of anticoagulant or drug abuse such as of amphetamine, and the one having most high frequency is hypertensive cerebral hemorrhage. In addition, cerebral hemorrhage is classified into traumatic and non-traumatic. Non-traumatic cerebral hemorrhage is generally said to be sudden one and occupies about 10% of stroke. The non-traumatic cerebral hemorrhage is further divided into symptomatic one whose bleeding source is clear and primary cerebral hemorrhage whose bleeding source is unclear. The symptomatic one is generated by the rupture of aneurysm or intracranial arteriovenous malformation, blood disease, bleeding tendency and the like. The primary cerebral hemorrhage includes hypertensive cerebral hemorrhage which is based on hypertension, a cerebral hemorrhage whose evident cause and basal disease cannot be pointed out (idiopathic cerebral hemorrhage) and the like.

Morbid states of cerebral hemorrhage at the acute phase are destruction of cerebral parenchyma by hematoma (primary brain lesion) and successively occurring secondary brain lesion in peripheral brain area. The brain tissue which was present in a region where hematoma progressed undergoes direct lesions such as breakage or deviation of axon due to compression of hematoma. In addition, nerve cells also undergo a secondary lesion accompanied by degeneration of the broken axon. Even in the case of a brain tissue which is adjacent to the hematoma but escaped from a direct lesion, brain edema and intracranial pressure of the whole brain are accelerated due to compression by the edema upon peripheral brain tissues so that it induces cerebral hernia (*Clinical Neuroscience,* 12 (12): 29 - 32, 1994).

It is said that hypertensive cerebral hemorrhage occupies about 60% of the bleeding in the cerebral parenchyma. Its main cause is hypertension, and the risk of bleeding becomes markedly high when arteriosclerosis is added to hypertension. Pathologically, it is considered that the bleeding occurs when vascular necrosis or small aneurysm is generated due to degeneration of cerebral vessel. In case that normal brain tissues are compressed and damaged due to acceleration of intracranial pressure caused by bleeding and further cause cerebral hernia, this is a serious disease because its mortality rate reaches a high frequency.

Onset of cerebral hemorrhage occurs during action accompanying disturbance of consciousness in many cases. The bleeding occurs in cerebral cortex, thalamus, cerebellum, pons and the like too, but the most frequent in this country is putaminal bleeding (*Clinical Neuroscience*, 12 (12): 29 - 32, 1994). Depending on the region where bleeding occurred and the size of bleeding, disturbance of consciousness, hemiplegia, aphasia and the like various neurological symptoms are developed (*Clinical Neuroscience*, 12 (12): 29 - 32, 1994). When hematoma is enlarged due to continuation of bleeding or re-bleeding, the neurological symptoms surely worsen and, in the case of profuse bleeding, 50% or more of the patients die within several days. In case that a patient escaped death, consciousness is restored as the overflowed blood is reabsorbed and the neurological symptoms are gradually alleviated. In general, a disorder remains to a certain degree and, in the case of dominant cerebral hemisphere disease, becomes aphasia.

Neurological symptoms of putaminal bleeding include rapidly appearing hemiplegia, sensory disturbance, homonymous hemianopsia, contralateral conjugate deviation, higher order cerebral function disease and headache, and it is said that the degree of hemiplegia is stronger than sensory disturbance (*Clinical Neuroscience*, 12 (12): 29 - 32, 1994).

Treatment of cerebral hemorrhage includes medical treatment and surgical treatment.

The medical treatment is carried out when hematoma is small or risk of operation is high. As the medical treatment, there are reports on hemodilution therapy, glycerol therapy and steroid therapy. These are therapies which aim at preventing expansion of bleeding and secondary brain lesion, but the effectiveness of medical treatment by these methods has not been proved.

The surgical treatment aims at reducing brain edema and secondary brain lesion around hematoma by removing the hematoma. A hematoma-removing craniotomy is carried out in the case of a large hematoma and subcortical bleeding or cerebellar bleeding, and a stereotaxic operation of cerebral hematoma suction is carried out on a medium to small hematoma deeply located in the brain for the purpose of stopping neuronal loss to the minimum level and improving functional prognosis. However, therapeutic result of the conservative treatment is good in comparison with the surgical treatment in lucid cases regardless of the region of cerebral hemorrhage. In addition, there is no operational adaptability in cases of deep coma because of poor prognosis for life and functions (*Diagnosis and Treatment* (written in Japanese), 89 (11): 2041 - 2045, 2001).

Thus, it is the present situation that there are no therapeutic agents and therapeutic methods which show sufficient efficacy for cerebral hemorrhage. Particularly, there is no therapeutic agent which aims at improving neurological symptoms caused by cerebral hemorrhage.

Zonampanel (YM 872) is the AMPA receptor antagonist (to be referred also to as AMPA antagonist hereinafter) disclosed in Patent Reference 1, namely [7-(1H-imidazol-1-yl)-6-nitro-2,3-dioxo-3,9-dihydroquinoxalin-1(2H)-yl]acetic acid monohydrate, which is shown to have potent and selective AMPA receptor binding activity and high water-solubility.

It has been reported that zonampanel has an effect to inhibit progress of infarction foci in various cerebral infarction models.

The AMPA receptor is expressed in neurons and glial cells. It is considered that when glutamic acid is excessively discharged into brain parenchyma caused by ischemia, the AMPA receptor is continuously stimulated to cause inflow of a large amount of calcium intracellularly, thereby damaging the cells. It has been shown using various cerebral infarction model animals that an AMPA antagonist or the like cerebroprotective agent inhibits neuronal death induced by ischemic cerebrovascular accidents such as cerebral infarction.

Since an AMPA antagonist or the like cerebroprotective agent has an action upon tissue lesion caused by ischemic reperfusion, a possibility has been suggested that it also has a protective action upon cerebrovascular accidents and can be used for cerebral hemorrhage and the like (Non-patent Reference 1).

However, bleeding did not occur in reality in the ischemic reperfusion model-aided tests so far reported. In addition, it is considered that the aforementioned suggestion that it could be used for cerebral hemorrhage is a suggestion for hemorrhagic cerebral infarction which is different as a disease from cerebral hemorrhage. That is, hemorrhagic changes appear in infarct among about 80% of cases during a period of from 1 to 3 weeks after onset of cerebral infarction (Non-patent Reference 2). While the hemorrhagic infarction causes petechial bleeding through the acceleration of permeability of vessel walls of micro blood vessels inside the brain due to ischemic changes and subsequent leaking of blood into the peripheral moiety of blood vessels, the cerebral hemorrhage accompanies vascular brain edema through the formation of hematoma which compresses its peripheral brain tissues.

In addition, though cell death in the periphery of hematoma after cerebral hemorrhage has not been elucidated yet, there is a report stating that caspase-3 is concerned therein (Non-patent Reference 3).

Thus, since cerebral hemorrhage and hemorrhagic infarction are different from each other clinically (by diagnostic imaging) and morpho-pathologically (Non-patent Reference 4), it is considered that cerebral hemorrhage and hemorrhagic infarction are different as diseases and the mechanism of neuronal death is also different.

Accordingly, the effect upon cerebral hemorrhage cannot be predicted based on the action to inhibit tissue lesion by ischemic reperfusion.

In addition, the neurological symptoms improving action of zonampanel in an ischemic reperfusion model is observed starting after 24 hours of the drug administration (Non-patent Reference 5), the neurological symptoms improving action upon cerebral hemorrhage confirmed by the present invention does not show significant effect 2 days after administration but shows significant improving effect 14 days after administration. This fact means that the effect of zonampanel upon neurological symptoms is an action which is different in ischemic reperfusion and cerebral hemorrhage.

In this connection, it has been reported that GYK152466 as one of the AMPA antagonists inhibits vascular permeability acceleration in a subarachnoid bleeding model animal injected with one's own blood (Non-patent Reference 6).

However, the aforementioned test suggests an effect of GYK152466 to inhibit brain edema because of its action to inhibit vascular permeability acceleration generated as a result of brain pressure acceleration caused by the blood injected into subarachnoid, but its action to improve neurological symptoms caused by subarachnoid bleeding. In addition, the neurological symptoms generated by subarachnoid bleeding are ones caused by disturbance of consciousness due to intracranial pressure or ischemic neuronal death due to vascular twitching which occurs at the chronic phase, but are not symptoms caused by the bleeding in the brain parenchyma.

As described in the above, to date there are no reports on the effect of zonampanel, a salt thereof or the like upon cerebral hemorrhage and on its effect to improve neurological symptoms accompanied by cerebral hemorrhage.

In addition, the action to improve neurological symptoms generated as a result of undergoing physical damage, degeneration or compression due to hematoma formed in the brain parenchyma cannot be expected different from the aforementioned effect of glutamic acid to prevent development of infarct foci caused by ischemic neuronal death.

[Patent Reference 1]
PCT international publication pamphlet WO 96/10023

[Non-patent Reference 1]
*Shindan to Chiryo*, 89 (11): 2017 - 2022, 2001

[Non-patent Reference 2]
*Shindan to Chiryo,* 89 (11): 2059 - 2064, 2001

[Non-patent Reference 3]
*Neurosurgery*, 48 (4): 875 - 883, 2001

[Non-patent Reference 4]
*Clinical Neuroscience,* 12 (12): 29 - 32, 1994

[Non-patent Reference 5]
*Neuropharmacology,* 39: 211 - 217, 2000

[Non-patent Reference 6]
*Brain Research Bulletin*, 45 (2): 163 - 166, 1998

### Disclosure of the Invention

An object of the present invention is to provide an agent for treating cerebral hemorrhage and an agent for improving neurological symptoms accompanied by cerebral hemorrhage, as novel uses of zonampanel or a salt or the like thereof.

With the aim of achieving the aforementioned object, the present inventors have conducted intensive studies and found unexpectedly that zonampanel or a salt or the like thereof accelerates improvement of neurological symptoms caused by cerebral hemorrhage, and thereby accomplished the following inventions.
(1) An agent for treating cerebral hemorrhage, which comprises [7-(1H-imidazol-1-yl)-6-nitro-2,3-dioxo-3,4-dihydroquinoxalin-1(2H)-yl]acetic acid or a salt or a hydrate thereof as an active ingredient.
(2) An agent for improving neurological symptoms accompanied by cerebral hemorrhage, which comprises [7-(1H-imidazol-1-yl)-6-nitro-2,3-dioxo-3,4-dihydroquinoxalin-1(2H)-yl]acetic acid or a salt or a hydrate thereof as an active ingredient.
(3) Use of [7-(1H-imidazol-1-yl)-6-nitro-2,3-dioxo-3,4-dihydroquinoxalin-1(2H)-yl]acetic acid or a salt or a hydrate thereof for manufacturing a medicament for treating cerebral hemorrhage which comprises a therapeutically effective amount of the same as an active ingredient.
(4) Use of [7-(1H-imidazol-1-yl)-6-nitro-2,3-dioxo-3,4-dihydroquinoxalin-1(2H)-yl]acetic acid or a salt or a hydrate thereof for manufacturing a medicament for improving neurological symptoms accompanied by cerebral hemorrhage which comprises a therapeutically effective amount of the same as an active ingredient.
(5) A method for treating cerebral hemorrhage, which comprises administering a composition comprising a therapeutically effective amount of [7-(1H-iznidazol-1-yl)-6-nitro-2,3-dioxo-3,4-dihydroquinoxalin-1(2H)-yl]acetic acid or a salt or a hydrate thereof and a pharmaceutically acceptable carrier to a patient.
(6) A method for improving neurological symptoms accompanied by cerebral hemorrhage, which comprises administering a composition comprising a therapeutically effective amount of [7-(1H-imidazol-1-yl)-6-nitro-2,3-dioxo-3,4-dihydroquinoxalin-1(2H)-yl]acetic acid or a salt or a hydrate thereof and a pharmaceutically acceptable carrier to a patient.

### Brief Description of the Drawings

Fig. 1: A graph showing change in the neurological symptom score in each administered group during 14 days after collagenase injection.
Fig. 2: A graph showing median of the neurological symptom score in each administered group on the 2nd day (left side of the graph) and the 14th day (right side of the graph) after collagenase injection.

### Best Mode for Carrying Out the Invention

Further description on the present invention is as follows.

Cerebral hemorrhage means intracerebral hemorrhage, namely a bleeding generated inside the brain parenchyma, and it includes ventricular bleeding but does not include subarachnoid and subdural bleeding. Cerebral hemorrhage is a phenomenon in which blood vessel wall is ruptured and blood is thereby leaked into outside moiety of the vessel, and a leakage of blood into outside moiety of the vessel caused by the increase of permeability of vessel wall (hemorrhagic cerebral infarction) is not included therein.

Depending on the bleeding regions, cerebral hemorrhage is classified into putaminal bleeding, thalamic bleeding, pontine (brainstem) bleeding, cerebellar bleeding, subcortical bleeding and the like. As the use of the present invention, preferred is a bleeding into striatum (a complex of nuclei comprising putamen, caudate nucleus and globus pallidum), more preferred are putaminal bleeding and caudate nucleus bleeding.

The agent for treating cerebral hemorrhage means an agent for inhibiting or improving various symptoms accompanied by cerebral hemorrhage, and illustratively, it means 1) effect of inhibiting or improving symptoms generated by the lesion of brain tissues caused by cerebral hemorrhage, particularly inhibition of nausea and vomiting, and the effect to inhibit or improve spasm, sensory disturbance, disturbance of consciousness, or difficulty of moving, speech disorder and the like neurological symptoms, and 2) effect of inhibiting increase of tissue destruction, and further 3) effect of preventing expression of brain edema caused by hematoma and/or expression of symptoms caused by brain edema, and the therapeutic agent of the present invention has at least one effect of the effects exemplified in the above.

Among the aforementioned effects of the agent for treating cerebral hemorrhage, the neurological symptoms accompanied by cerebral hemorrhage mean symptoms due to disorder of neuronal function controlled by a region where the brain received a lesion by cerebral hemorrhage, and illustratively, they are neurological symptoms caused by putaminal bleeding and caudate nucleus bleeding. As the symptoms due to disorder of neuronal function controlled by a region where the brain received a lesion by cerebral hemorrhage, they illustratively mean difficulty of moving, speech disorder, sensory disturbance, ophthalmic disorder and the like.

The difficulty of moving means a state in which voluntary movement is difficult or impossible to perform or cannot be carried out smoothly due to the brain lesion caused by cerebral hemorrhage, and its examples include motor paralysis and ataxia. Preferred is the improvement of motor paralysis.

The motor paralysis means a state in which voluntary movement is difficult or impossible to perform due to damage on the motor center by cerebral hemorrhage. Included in the motor paralysis are hemiplegia, paraplegia, quadriplegia, monoplegia and the like having spastic or flaccid paralysis. Preferred is the improvement of hemiplegia.

The ataxia means a state in which voluntary movement cannot be carried out smoothly due to disorder of mutual valance and cooperative movement between muscle groups in spite of the absence of muscular strength reduction and paralysis.

The disturbance of consciousness means mainly reduction of consciousness level, and means a state in which reaction cannot be made based on a judged result by correctly recognizing a stimulus from the outside. The disturbance of consciousness includes syncope, eye inclination, stupor, semi-coma and coma as changes of consciousness lucidity, and delirium, twilight state and the like as changes of consciousness contents.

The speech disorder includes dysarthria and aphasia.

The dysarthria is caused by a disorder of articulation-related muscle groups or of nervous system controlling the former, and generally expressed as "cannot speak clearly" or "tongue is getting thick". The dysarthria includes a paralytic one and those caused by disorders of cerebellum and extrapyramidal tract system, and the disorders by cerebral hemorrhage are caused by disorders of pyramidal tract and cranial nerves.

The aphasia is a state in which correct speaking and understanding of words are troubled due to disorders of speech region in the brain, and occurs independent of the presence or absence of the difficulty of moving of the articulation organ. In most cases, this is caused by disorders of the left side cerebral hemisphere. The aphasia includes Broca aphasia, Wernicke aphasia, total aphasia, conduction aphasia and the like.

The sensation includes tactile sense, sense of pressure, temperature sensation and the like superficial sensations, position sensation, vibratory sensation and the like deep sensibilities and two-point discrimination, skin writing sensations and the like complex sensations.

The sensory disturbance is a state in which these sensations are not normally recognized due to disorders of the brain, and includes anesthesia (deletion), hypesthesia (decline), hyperesthesia and abnormal sensation (paresthesia), depending on the degree of the state. In addition, this is classified also by the regions where sensory disturbance in one side of the body, superficial sensory disturbance, total sensory disturbance and the like sensory disturbances are generated.

Examples of the ophthalmic disorder include defect of visual field, narrowing of visual field, hemianopsia, ptosis, narrowing of pupil, dilatation of pupil, nystagmus, horizontal gaze palsy, vertical gaze palsy, oculomotor paralysis of eye ball, abducens palsy, horizontal conjugate deviation, strabismus, eyeball position abnormality, Honer symptom and the like.

Examples of the neurological symptoms caused by putaminal bleeding include hemiplegia in the opposite side to focal side (spastic), sensory disturbance in one side of the body, crossed homonymous hemianopsia, eyeball conjugate deviation to focal side, aphasia, apraxia, agnosia and the like.

Examples of the neurological symptoms caused by caudate nucleus bleeding include focal side opposite side hemiplegia, atony in one side of the body, hypesthesia, nuchal rigidity and the like.

The improvement of neurological symptoms accompanied by cerebral hemorrhage means an effect in which the improving degree of the aforementioned neurological symptoms exceeds a case of not administering the compound to be used in the present invention, or an effect in that the period of time required for recovering the aforementioned neurological symptoms is shortened in comparison with a case of not administering the compound to be used in the present invention.

It is desirable to start administration of the compound to be used in the present invention during a period of from immediately after the onset of cerebral hemorrhage to 48 hours thereafter, for the purpose of improving symptoms of cerebral hemorrhage.

In addition, the use of the present invention includes therapeutic or improving effects on all of the aforementioned symptoms regarding cerebral hemorrhage.

Zonampanel forms salt with an acid or base.

### Preferred is a pharmaceutically acceptable salt.

Examples of the salt with an acid include acid addition salts with inorganic acids such as hydrochloric acid, hydrobromic acid, hydroiodic acid, sulfuric acid, nitric acid, phosphoric acid and the like mineral acids and the like, and with organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, ethanesulfonic acid, glutamic acid and the like. Examples of the salt with a base include salts with sodium, potassium, magnesium, calcium, aluminum and the like inorganic bases, methylamine, ethylamine, ethanolamine and the like organic bases, or lysine, arginine, ornithine and the like basic amino acids, and ammonium salts. In addition, it can form a hydrate, a solvate with ethanol or the like and crystal polymorphism.

### Preferred is [7-(1H-imidazol-1-yl)-6-nitro-2,3-dioxo-3,4-dihydroquinoxalin-1(2H)-yl]acetic acid monohydrate.

It is possible to use the therapeutic agent of the present invention and other medicament together. For example, zonampanel or a salt or the like thereof can be administered alone or together with other medicament or nutrient. Illustratively, there are hemodilution therapy, glycerol therapy, steroid therapy, agents for treating cerebral aneurysm rupture, rupture of intracranial arteriovenous malformation, cavernous angioma, head injury and amyloid angiopathy, anticoagulants, amphetamine and the like, as well as agents which are administered for the treatment of brain edema, migraine, brain tumor, cerebral infarction and the like, and their examples include warfarin, heparin, aspirin, sumatriptan succinate, solmitriptan, interferon beta, nimustine hydrochloride, alteplase, tisokinase, liteplase, nateplase, pamiteplase, monteplase, lanoteplase, tenecteplase, edalabon, ozagrel sodium, citicoline, argatroban, glyceol, mannitol, CDP-choline and the like.

The pharmaceutical preparation which contains one or two or more of the compounds or salts thereof of the present invention as the active ingredient is prepared using a carrier or a filler generally used in making pharmaceutical preparations and other additives.

Regarding the carrier or filler for use in the pharmaceutical preparation may be either solid or liquid, and its examples include lactose, magnesium stearate, starch, talc, gelatin, agar, pectin, gum arabic, olive oil, sesame oil, cacao butter, ethylene glycol and the like and other generally used substances.

Its administration may be either by oral administration through tablets, pills, capsules, granules, powders, solutions or the like or by parenteral administration through injections for intravenous injection, intramuscular injection or the like, suppositories, percutaneous preparations or the like.

Clinical dose is optionally decided by taking into consideration symptoms, age, sex and the like of each patient to be treated, but is generally from 100 to 2,000 mg, preferably about 900 mg, per day per adult. The daily dose of from 100 to 2,000 mg per adult may be administered once a day or by dividing it into 2 to 4 doses. In the case of intravenous administration or continuous intravenous administration, it may be administered by 1 hour to 24 hours per day.

As described in the foregoing, the dose is decided depending on various conditions. When effective, a dose smaller than the above range can be used.

The compound to be used in the present invention can be administered through various administration forms, mainly by parenteral administration, illustratively by subcutaneous injection, intramuscular injection, intravenous injection, percutaneous injection, intraspinal injection, epidural injection or intraarticular injection, or by topical application or, when possible, by oral administration.

The injections for parenteral administration include aseptic aqueous or non-aqueous solutions, suspensions and emulsions. Examples of the diluent for use in the aqueous solutions and suspensions include distilled water for injection and physiological saline. Examples of the diluent for use in the non-aqueous solutions and suspensions include propylene glycol, polyethylene glycol, olive oil or the like plant oil, ethanol or the like alcohol, polysorbate 80 (trade name) and the like. Such a composition may further contain additive agents such as an antiseptic, a moistening agent, an emulsifying agent, a dispersing agent, a stabilizing agent (e.g., lactose) and a solubilization assisting agent (e.g., meglumine). These are sterilized for example by filtration through a bacteria retaining filter, blending of a germicide or irradiation. Alternatively, these may be used by producing sterile solid compositions and dissolving them in sterile water or a sterile solvent for injection prior to their use.

The solid composition for use in the oral administration according to the present invention is used in the form of tablets, powders, granules and the like. In such a solid composition, one or more active substances are mixed with at least one inert diluent such as lactose, mannitol, glucose, hydroxypropylcellulose, microcrystalline cellulose, starch, polyvinyl pyrrolidone or aluminum magnesium silicate. In accordance with the usual way, the composition may contain other additives than the inert diluent, such as magnesium stearate or the like lubricant, calcium cellulose glycolate or the like disintegrating agent, lactose or the like stabilizing agent and glutamic acid, aspartic acid or the like solubilization assisting agent. As occasion demands, tablets or pills may be coated with a sugar coating or a film of a gastric or enteric substance such as sucrose, gelatin, hydroxypropylcellulose, hydroxypropylmethylcellulose phthalate or the like.

The liquid composition for oral administration includes pharmaceutically acceptable emulsions, solutions, suspensions, syrups, elixirs and the like and contains a generally used inert diluent such as purified water or ethanol. In addition to the inert diluent, this composition may also contain a moistening agent, a suspending agent and the like auxiliary agents, as well as sweeteners, flavors, aromatics and antiseptics.

### [Examples]

Next, the present invention is described further in detail based on examples, but the invention is not limited to these examples.

Therapeutic affects of the present invention were confirmed by the following test methods.

### Test Examples

### 1. Preparation of cerebral hemorrhage model

A cerebral hemorrhage model was prepared by the following procedure by adding a partial modification to the method of Rosenberg *et al*. (*Stroke,* 21, 801 - 807, 1990). Each of male Sprague-Dawley rats (240 to 330 g in body weight, Charles River Japan) was subjected to continuous anesthesia with 1% halothane under spontaneous respiration and fixed on a stereotaxic instrument. The skull was exposed by carrying out median skin incision of the head of the animal, and a small bone window was opened on the skull using a dental drill (C730, MINIMO) at a position of 0.2 mm in the front side (A: +0.2 mm) and 3.0 mm in the left side (L: +3.0 mm) based on Bregma in accordance with the Paxinos's brain pictorial book (Paxinos G and Watson C, The rat brain in the stereotaxic coordinates (1986) Academic Press, New Yolk). An administration needle of 30G was inserted to a depth of 6.0 mm (H: -6.0 mm) from the skull and then pulled out 0.3 mm to ensure an administration space (central part of the striatum). A solution of collagenase (10 units/ml, type IV, Lot 99H8641, Sigma) was injected at a flow rate of 0.2 µl/min for 7 minutes (1.4 µl) using a micro-syringe pump (Neuroscience Syringe Pump 100, Muromachi). The administration needle was slowly pulled out after completion of the injection, scalp suture was carried out, and then the animal was revived by warming up under an incandescent lamp. It has been reported that a cerebral hemorrhage locally limited to the striatum is generated 0.5 to 4 hours after injection of collagenase in this rat cerebral hemorrhage model (*Stroke*, 27, 2312 - 2320, 1996). Also, it is known that an animal which caused bleeding in the striatum shows a turning behavior (a neurological symptom) to the opposite side (right side) of the hematoma forming side (left side of the striatum) (*Stroke*, 21, 801 - 807, 1990, *Stroke*, 27, 2312 - 2320, 1996).

### 2. Administration of medicament

As the administration of medicament, zonampanel monohydrate (hereinafter, compound a) was used. After 30 minutes of the collagenase injection, intravenous injection of zonampanel was started via a canula indwelled in the common jugular vein. The compound a was dissolved in physiological saline at a concentration of 10 mg/5 ml or 20 mg/5 ml and then subjected to 24 hours of intravenous continuous injection under shading at a dose of 10 mg/5 ml/kg/h (compound a (10 mg/kg) administration group) or 20 mg/5 ml/kg/h (compound a (20 mg/kg) administration group). In the control group, physiological saline was subjected to 24 hours of intravenous continuous injection at a dose of 5 ml/kg/h (physiological saline administration group). The number of animals in respective groups was 6 animals in the compound a (10 mg/kg) administration group, 9 animals in the compound a (20 mg/kg) administration group, and 9 animals in the physiological saline administration group.

### 3. Neurological symptom

Staring after 30 minutes of the injection of collagenase into the striatum, the compound a was subjected to 24 hours of intravenous continuous injection at a dose of 10 mg/kg/h (compound a (10 mg/kg) administration group, 6 cases) or 20 mg/kg/h (compound a (20 mg/kg) administration group, 9 cases), and a neurological symptom of each animal was observed before the injection of collagenase and on the 2nd and 14th days after the collagenase injection. Physiological saline was administered to the control group (physiological saline administration group, 9 cases).

Scoring criteria of the neurological symptom are as follows. This was scored by 5 steps (0 to 4) based on the behavior of each animal when the waist part of the animal was pushed toward the front side.

Score 4: Does not move or hardly moves.

Score 3: Turns right, does not go straight on or turn left.

Score 2: Turning right is predominant but sometimes go straight on, does not turn left.

Score 1: Turning right is predominant but sometimes turns left.

Score 0: Normal, no left-right turning inclination.

### 4. Statistical method

Statistical test of the neurological symptom was carried out by Steel test, regarding the scores of the drug administered groups on the 2nd and 14th days after collagenase administration using the physiological saline administration group as the control. The value of p < 0.05 was judged significant.

### Results

### (1) Change of neurological symptom scores

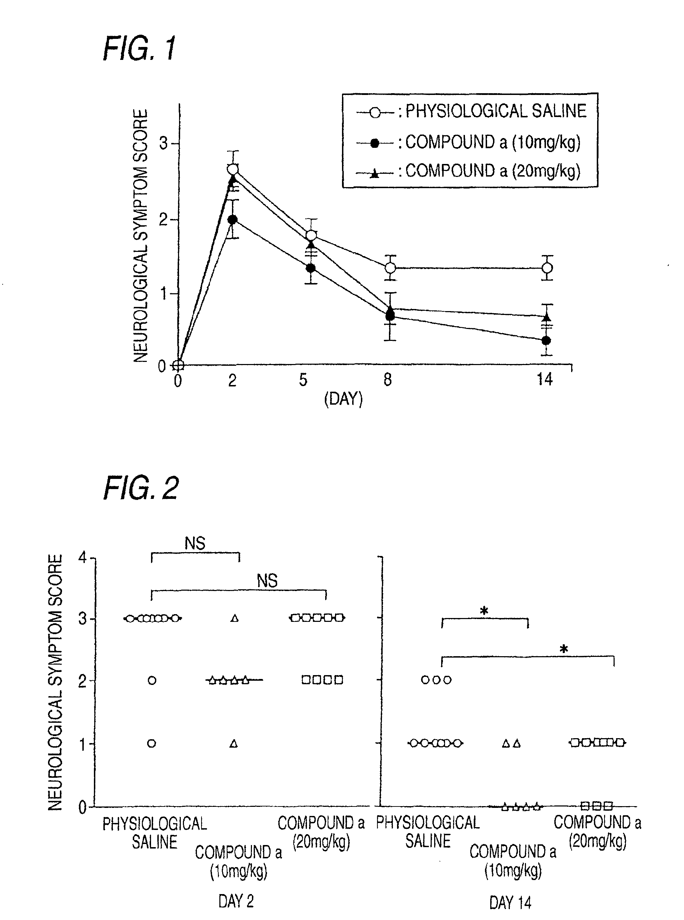

Results of the neurological symptom scores before collagenase injection (0th day) and 2nd, 5th, 8th and 14th days after its injection are shown in Fig. 1.

The values of Fig. 1 show change of average neurological symptom score value ± standard deviation in each administration group.

The values of Fig. 2 show the number of animals and median value of each score, which were compared on the 2nd day (left side of the graph) and the 14th day (right side of the graph) after collagenase injection. The symbol * shows that the value has a significance (p< 0.05) against the physiological saline administration group, and NS shows no significance (steel test).

The neurological symptom before the collagenase injection was normal (score 0). Each of the physiological saline administration group, compound a (10 mg/kg) administration group and compound a (20 mg/kg) administration group showed the most heavy neurological symptom on the 2nd day after collagenase injection, and the compound a administration groups showed an improving tendency in comparison with the control as the days elapsed.

### (2) Comparison of neurological symptom scores on the 2nd day and 14th day after collagenase injection

A graph showing median values of the neurological symptom scores in each administration group on the 2nd day (left side of the graph) and the 14th day (right side of the graph) after collagenase injection is shown in Fig. 2.

The neurological symptom scores in respective groups on the 2nd day were almost the same (left side of Fig. 2), but the neurological symptom on the 14th day was significantly light in the compound a administration groups in comparison with the physiological saline administration group (right side of Fig. 2).

In the cerebral hemorrhage model used in the present invention, bleeding occurs in the striped body (a complex nervous nucleus comprising putamen, caudate nucleus and globus pallidum). Accordingly, hemiplegia as one of the difficulties of moving is significant so that a neurological symptom originated from hemiplegia is scored.

Thus, it was confirmed that the compound has the effect to improve neurological symptoms accompanied by cerebral hemorrhage, particularly hemiplegia, on the 14th day after injection of collagenase.

### Example (Production of freeze-dried pharmaceutical preparations)

A 33.3 g portion of meglumine was dissolved in 400 ml of water for injection, and 10 g of the compound a was added thereto and dissolved with stirring. A 1,400 ml portion of water for injection was added to this solution, 40 g of mannitol was dissolved therein, and then the total volume was adjusted to 2,000 ml by adding water for injection. This solution was sterilized by filtration in the usual way and then packed in 15 ml portions into 30 ml capacity vials and freeze-dried in the usual way to obtain freeze-dried pharmaceutical preparations of the compound to be used in the present invention.

### Industrial Applicability

According to the present invention, zonampanel or a salt or the like thereof shows an effect to improve cerebral hemorrhage and neurological symptoms accompanied by cerebral hemorrhage and therefore is useful as an agent for treating cerebral hemorrhage.

## Claims

1. An agent for treating cerebral hemorrhage, which comprises [7-(1H-imidazol-1-yl)-6-nitro-2,3-dioxo-3,4-dihydroquinoxalin-1(2H)-yl]acetic acid or a salt or a hydrate thereof as an active ingredient.

2. An agent for improving neurological symptoms accompanied by cerebral hemorrhage, which comprises [7-(1H-imidazol-1-yl)-6-nitro-2,3-dioxo-3,4-dihydroquinoxalin-1(2H)-yl]acetic acid or a salt or a hydrate thereof as an active ingredient.

3. Use of [7-(1H-imidazol-1-yl)-6-nitro-2,3-dioxo-3,9-dihydroquinoxalin-1(2H)-yl]acetic acid or a salt or a hydrate thereof for manufacturing a medicament for treating cerebral hemorrhage which comprises a therapeutically effective amount of the same as an active ingredient.

4. Use of [7-(1H-imidazol-1-yl)-6-nitro-2,3-dioxo-3,4-dihydroquinoxalin-1(2H)-yl]acetic acid or a salt or hydrate thereof for manufacturing a medicament for improving neurological symptoms accompanied by cerebral hemorrhage which comprises a therapeutically effective amount of the same as an active ingredient.

5. A method for treating cerebral hemorrhage, which comprises administering a composition comprising a therapeutically effective amount of [7-(1H-imidazol-1-yl)-6-nitro-2,3-dioxo-3,4-dihydroquinoxalin-1(2H)-yl]acetic acid or a salt or a hydrate thereof and a pharmaceutically acceptable carrier to a patient.

6. A method for improving neurological symptoms accompanied by cerebral hemorrhage, which comprises administering a composition comprising a therapeutically effective amount of [7-(1H-imidazol-1-yl)-6-nitro-2,3-dioxo-3,4-dihydroquinoxalin-1(2H)-yl]acetic acid or a salt or a hydrate thereof and a pharmaceutically acceptable carrier to a patient.
